# EUROPEAN PATENT SPECIFICATION

(11) **EP 4 566 560 B1**
(45) Date of publication and mention of the grant of the patent: **18.11.2026**
(21) Application number: 23854133.8
(22) Date of filing: 30.06.2023
(51) Int. Cl.: A61B 34/20, A61B 90/00, A61B 46/20

(54) **MULTIFUNCTIONAL THIN FILM FOR SURGERY**
MULTIFUNKTIONALE DÜNNSCHICHT FÜR DIE CHIRURGIE
FILM MINCE MULTIFONCTIONNEL POUR UNE CHIRURGIE

(30) Priority: 18.08.2022 CN 202210991491
(43) Date of publication of application: 11.06.2025
(73) Proprietor: Agloe Medical Technology Co., Ltd., Suzhou, Jiangsu 215413 (CN)
(72) Inventor: YOUNG, Victor, Suzhou, Jiangsu 215413 (CN); SHI, Weisong, Suzhou, Jiangsu 215413 (CN); CHEN, Chaoliang, Suzhou, Jiangsu 215413 (CN); SUI, Guangyu, Suzhou, Jiangsu 215413 (CN)
(74) Representative: Santarelli
(86) International application number: PCT/CN2023/104815
(87) International publication number: WO 2024/037228

(56) References cited:
- CN-A- 112 086 012
- CN-A- 114 469 375
- CN-A- 115 486 936
- CN-U- 210 077 877
- CN-U- 219 579 023
- US-A1- 2003 182 815
- US-A1- 2003 184 081
- US-A1- 2003 187 458
- US-A1- 2015 223 906
- US-A1- 2022 226 069

## Description

This application claims the priority to Chinese Patent Application No. 202210991491.5 filed with the Chinese Patent Office on August 18, 2022 and entitled "MULTIFUNCTIONAL THIN FILM FOR SURGERY".

### Technical field

The present invention relates to the field of surgical imaging, and in particular, to a multifunctional thin film for surgery.

### Background

In surgical operations based on surgical robots, imaging devices (surgical robots) are combined to identify and locate the opening of the patient. However, during the surgery, the method of extracting wound location information based on image processing is often interfered by many external factors. For example, the lighting factor may cause unclear imaging of the opening position, and the opening position may be blocked. Consequently, the camera is unable to accurately locate the opening position, and it is not conducive to the smooth progress of the surgery.

US 2015/223906 A1 relates to a medical procedure localization aid which produces reference marks on both the patient and the medical imaging scan. The aid is defined by an imaging substrate having indicia on one side that is opaque to medical imaging and a second substrate having indicia that appears on the patient's surface for the procedure. Anchor points align the second substrate to the same position as the first substrate on the patient. By visualizing the location of a target on the scan image relative to the indicia on the scan image and comparing that with indicia on the patient, a medical processional may reliably locate where a medical procedure should be performed.

CN 114 469 375 A relates to a developable skin positioning protective film, which comprises a positioner provided with an adhesive film material and a QR code scanning area. A scale table is printed on the surface of the positioner, and warps and wefts of the scale table are coatings which can be clearly developed by perspective of an X-ray machine with strong ray absorption. The film is smooth in surface, high in adhesive force and accurate in positioning.

US 2003/182815 A1 relates to a surgical site marking system for marking, correlating, and verifying that the surgical site which is to undergo the surgical procedure has been identified as the correct surgical site. The system utilizes labels and a series of checks to associate the patient with the correct surgery and appropriate surgical site.

### Summary

In view of this, the present invention provides a multifunctional thin film for surgery, which can solve the problem of accurate positioning of a surgical area by a surgical robot during surgery, and integrates preoperative and intraoperative data information, etc., to facilitate the management of surgical data.

In order to solve the above technical problem, the present invention provides a multifunctional thin film for surgery by a surgical operator as defined in claim 1. Embodiments of the invention are recited in the dependent claims. The multifunctional thin film includes a thin film main body, where the thin film main body is provided with a window that is used for surgical operation and that penetrates both ends in a thickness direction, and the thin film main body is provided with at least three layers of structure in the thickness direction, and the three layers of structure include:
three layers of structure in the thickness direction, and the three layers of structure include:
a thin film layer;
an identification layer, where the identification layer is provided on a surface of the thin film layer, and is located at the periphery of the window, and the identification layer is used to identify a position of the window and basic information related to the surgery; and
an isolation paper layer, where the isolation paper layer is provided on a surface of the identification layer away from the thin film layer.

According to one embodiment of the present invention, the identification layer includes:
a first identifier, where the first identifier is provided in a first area of the identification layer, and is used to identify and manage the basic information related to the surgery;
a second identifier, where the second identifier is provided in a second area of the identification layer, and is used to identify position coordinates of the window.

According to one embodiment of the present invention, the basic information related to the surgery includes: the time of the surgery, the location of the surgery, and data information of preoperative and intraoperative tests of a patient.

According to one embodiment of the present invention, the periphery of the window includes a top edge, a bottom edge opposite to the top edge, and two side edges respectively connecting the two ends of the top edge and the bottom edge, and the first identifier is provided on the top edge; and the second identifier is provided on at least two of the two side edges and the bottom edge.

According to one embodiment of the present invention, the first identifier and the second identifier are both two-dimensional barcode identifiers.

According to one embodiment of the present invention, the two-dimensional barcode identifier is a QR code or an ArUco code.

According to one embodiment of the present invention, a plurality of first identifiers are provided, a plurality of second identifiers are provided, and a spacing between the identifiers is greater than a preset spacing.

According to one embodiment of the present invention, the preset spacing is 2mm.

According to one embodiment of the present invention, each first identifier is provided with a corresponding first number according to a set position, and each second identifier is provided with a corresponding second number according to a set position.

According to one embodiment of the present invention, a size of the first identifier is greater than a size of the second identifier.

According to one embodiment of the present invention, the thin film layer, the isolation paper layer, and the identification layer are fixed by using an adhesive.

The beneficial effects of the above technical solution of the present invention are as follows:
For the multifunctional thin film for surgery according to the embodiments of the present invention, a window is provided on the thin film, it is convenient to perform surgical operation by using the window, and the window can also serve as a virtual reality projection window during the surgery. The periphery of the window is provided with an identifier, and combined with the positioning of the identification layer, a projection image of the surgical area can be displayed in real time, so that it is convenient for the camera to accurately locate the opening position of the patient according to the identifier, thereby solving the problem of line-of-sight obstruction during surgery. At the same time, by the setting of the identifier, a data management entrance is provided, and the data in the database can be queried and retrieved, so that more reliable data is provided for camera imaging, and a good foundation is established for the imaging system to better perform imaging on the intraoperative image.

### Brief description of drawings

FIG. 1 is a schematic structural diagram of a multifunctional thin film for surgery according to an embodiment of the present invention;
FIG. 2 is a schematic diagram of a three-dimensional structure of a multifunctional thin film for surgery according to an embodiment of the present invention; and
FIG. 3 is a schematic structural diagram of window imaging of a multifunctional thin film for surgery according to an embodiment of the present invention.

### Reference signs

Multifunctional thin film for surgery 100;
Thin film main body 10; Window 20; Image 30; Affected area 40;
Thin film layer 11;
Identification layer 12; First identifier 121; Second identifier 122;
Isolation paper layer 13.

### Detailed description of embodiments

In order to make the objective, technical solution and advantages of the embodiments of the present invention clearer, the technical solution of the embodiments of the present invention is clearly and completely described with reference to the drawings of the embodiments of the present invention. Obviously, the described embodiments are some of the embodiments of the present invention, not all of the embodiments. Based on the described embodiments of the present invention, all other embodiments obtained by ordinary technicians in the art belong to the scope of protection of the present invention.

The multifunctional thin film for surgery 100 of the embodiment of the present invention is described below in detail with reference to the drawings.

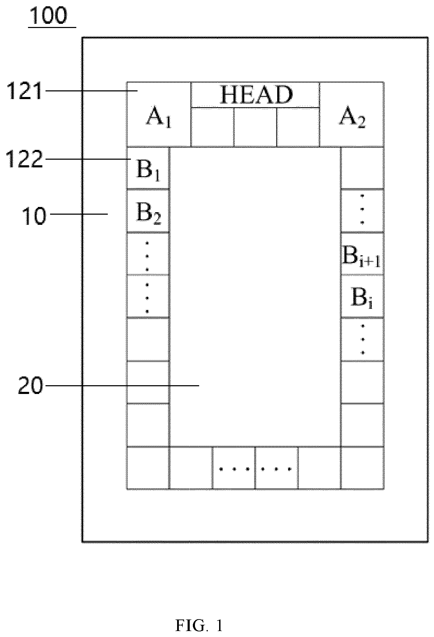

As shown in FIGS. 1 to 3, the multifunctional thin film for surgery 100 of the embodiment of the present invention includes a thin film main body 10 and a window 20.

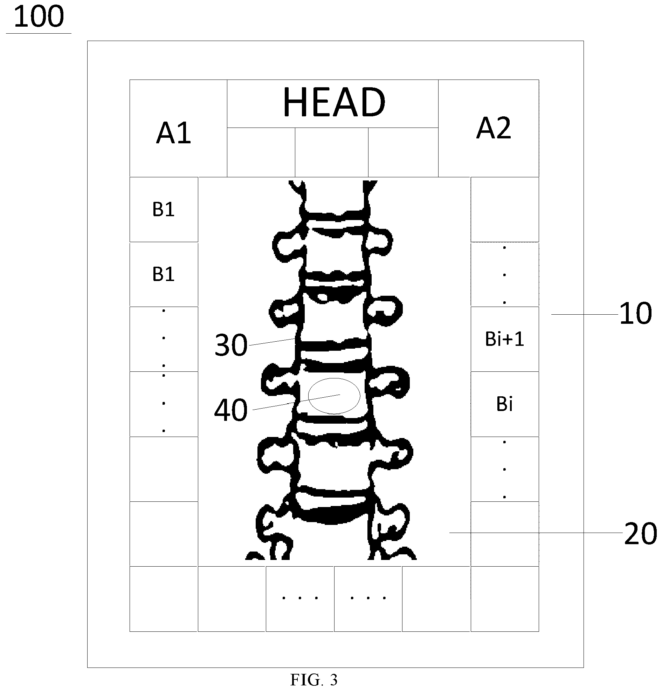

Specifically, the window 20 is provided on the thin film main body 10 and runs through a thickness direction of the thin film main body 10. The window can be a hollow structure or a non-hollow structure obtained by dividing on a layer of thin film. During the surgery, the operator can tear off the thin film in the window area obtained by dividing according to a preset trajectory, or can obtain a required size by cutting by using a knife according to the required size. The thin film main body 10 is provided with at least three layers of structure in a thickness direction thereof. As shown in FIG. 3, the thin film main body 10 includes a thin film layer 11, an identification layer 12, and an isolation paper layer 13. The thin film layer 11 and the isolation paper layer 13 are respectively provided on the surfaces of both sides of the identification layer 12. The thin film layer 11 is a transparent and thin plastic material, and light can pass through the thin film layer 11 to irradiate the identification layer 12. The identification layer 12 is used to identify the position of the window 20 and basic information related to the surgery. The isolation paper layer 13 has a certain hardness, which can ensure that the thin film as a whole remains flat, and prevent the identification layer 12 from winkling that affects the recognition of the identifier by subsequent imaging device. In addition, after the isolation paper layer 13 is separated from the identification layer 12, the identification layer 12 can be fixed on the periphery of the opening of the patient when being applied to the skin surface.

According to the multifunctional thin film for surgery 100 of the embodiment of the present invention, the window 20 is opened on the thin film, and the window 20 serves as a surgical operation window 20. For example, during the surgery, the side of the surgical thin film 100 with the thin film layer 11 is applied around the surgical opening of the patient, and the position of the opening of the patient is exposed to the surgical operator through the window 20, so that the operator can observe and perform operations on the tissue conditions inside the opening through the window 20. In addition, the window 20 is used as an intraoperative virtual reality projection window 20 to display a projection image of the surgical area in real time.

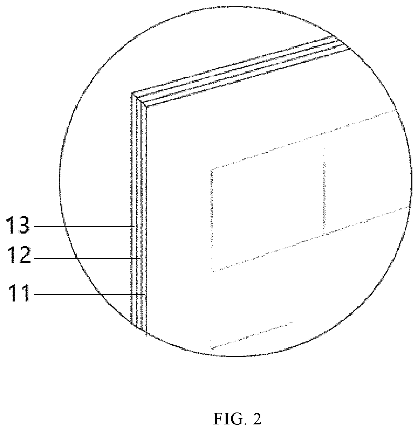

Refer to the schematic diagram of the window projection shown in FIG. 2. During the actual surgical process, the imaging device accurately locates the surgical site by using the identification layer 12, and then projects an image 30 (such as a preoperative CT image, or an intraoperative OCT image) into the window 20 by using a virtual reality technology. During projection, projection can be performed according to the actual proportion, the surgical area can be accurately positioned, and the operator can timely understand the location and depth information of the affected area 40 of the current surgery based on the projected image. When an imaging angle of the imaging device changes, the projected image is also updated in real time, so that convenience is provided for the surgery.

**In** the present invention, the imaging device can scan an identification code on the identification layer 12, and then can accurately locate the position of the opening of the patient by using the position of the identification code, so that the problem that the intraoperative imaging device (surgical robot) cannot accurately locate the surgical area is solved. **In** addition, the identification code can also be used as an identifier of a database entry. After the imaging device scans the identification code, the relevant data of the corresponding surgery can be retrieved by using the information provided by the identification code, so that the precise management of the surgical data by the imaging device is implemented. The thin film of the present invention can be combined with the virtual reality technology to provide more solutions for surgical operations and surgical robot system operations.

It should be noted that, in the above embodiments, the specific data information related to the surgery is only an exemplary description. In some embodiments, other data may be included, such as basic information about the surgical team members. This is not limited in the present invention.

According to one embodiment of the present invention, the identification layer 12 includes a first identifier 121 and a second identifier 122. The first identifier 121 is provided in a first area of the identification layer 12, and is used to identify the basic information related to the surgery. For example, the basic information related to the surgery can be the current surgical data, time, location, etc., and can also be associated with preoperative data and intraoperative data. For example, the preoperative data can include CT, MRI**,** X-ray images, etc. The intraoperative data can include C-arm machine images, OCT images, visible light imaging data, electromagnetic navigation data, and mechanical feedback data of the robot arm, etc. The data can be accurately managed after the surgery.

In the embodiment of the present invention, the second identifier 122 is provided in a second area of the identification layer 12, and is used to identify the position of the window 20. During the surgical process, the imaging device needs to perform imaging on and locate the opening position of the patient. During the imaging process, the lighting factor may cause the opening position to be unclear, and when the surgical operator moves the hand position, the opening position of the patient may be blocked, which causes the imaging device to be unable to track the opening position normally, affecting the precise positioning and imaging of the opening position. Therefore, by adding the second identifier 122 to the thin film main body 10, the second identifier 122 is easier to recognize, which is conducive to the precise positioning of the imaging device. In this way, through the cooperation of the first identifier 121 and the second identifier 122, not only the precise positioning of the opening position is implemented, but also the preoperative and intraoperative data of the surgery can be accurately managed, so that it is conducive to the smooth progress of the surgery.

In some embodiments, as shown in FIG. 1, the periphery of the window 20 includes a top edge, a bottom edge opposite to the top edge, and two side edges respectively connecting the two ends of the top edge and the bottom edge. The first identifier 121 is set on the top edge, and the top edge can be marked with an identifier "HEAD". After the identifier "HEAD" is attached on the top edge, the top edge can be attached to the corresponding position according to the head information. In addition, the first identifier 121 is set on the top edge (corresponding to the set position), which can also play a positioning role, that is, when the imaging device recognizes the first identifier 121 or the identifier HEAD, it can be determined that the position is the top edge position. In some embodiments, as shown in FIG. 1, a number (first number) can be set on the first identifier 121, for example, "A1" and "A2" in FIG. 1, so that the imaging device can identify the position of the first identifier 121 according to the number, and implement the preliminary positioning of the window position.

As shown in FIG. 1, the second identifier 122 is provided on at least two of the two side edges and the bottom edge, preferably, can be provided on the two side edges and the bottom edge (set position). By setting the second identifier on multiple edges, the second identifier 122 surrounds the opening position, so that it is more conducive to the accurate positioning of the opening position. After the imaging device recognizes the second identifier 122, the position of the opening can be known in time, so that it is conducive to the accurate positioning and shooting of the opening position by the imaging device.

In some embodiments, a number (a second number) can also be set in the second identifier 122. As shown in FIG. 1, when the first second identifier 122 on the left side edge of the frame near the first identifier 121 is used as B1, encoding is performed in order, B1, B2...Bi+3 until the second identifier 122 near the first identifier 121 on the right is arranged. When the imaging device recognizes the number B1, according to the position information of B1 in the frame of the thin film main body 10, for example, the first second identifier 122 on the left, and according to the quantity of second identifiers 122 in each edge, for example, there are eight second identifiers 122 on the left side edge, four second identifiers 122 on the bottom edge, and eight second identifiers 122 on the right side edge, the imaging device can obtain the position of the entire frame by calculation. Based on the above information, the imaging device can obtain the position of the entire frame by calculation, and then obtain the position of the opening, and timely perform positioning and shooting or accurately perform projection. In this way, even if the opening part is blocked during the surgery, or even most of the thin film is blocked, the imaging device can obtain the position of the opening by using at least one second identifier 122, so that the accurate positioning of the opening position is implemented, and the problem that the opening position cannot be accurately positioned due to the blocking of the opening position during the surgery is avoided.

Preferably, the first identifier 121 and the second identifier 122 are both two-dimensional barcode identifiers, for example, may be QR codes or ArUco codes.

In some embodiments, when there is a plurality of first identifiers 121 and a plurality of second identifiers 122, a case in which some two-dimensional barcodes cannot be accurately recognized due to skin deformation, affecting the positioning function can be prevented. The probability of unrecognition can be reduced by using a plurality of two-dimensional barcodes. In addition, a spacing is provided between the plurality of identifiers (two-dimensional barcodes), for example, 0.5mm, 1mm, 1.5mm, etc. Preferably, the spacing can be greater than 2mm, and bending may occur at the two-dimensional barcode spacing when the skin surface is uneven, thereby avoiding the bending of the two-dimensional barcode that affects the speed and accuracy of recognition.

According to one embodiment of the present invention, the size of the first identifier 121 is larger than the size of the second identifier 122, so that the imaging device can accurately obtain the first identifier 121, quickly understand the storage information corresponding to the first identifier 121, and quickly obtain the head of the thin film main body 10, so that it is conducive to the imaging device to quickly locate the initial position of the thin film as a whole.

In one embodiment of the present invention, the thin film layer 11, the identification layer 12, and the isolation paper layer 13 are fixed by using an adhesive, the connection method is simple, and it is conducive to the firm connection between the thin film layer 11 and the identification layer 12, and the rapid separation of the isolation paper layer 13 and the identification layer 12.

According to the multifunctional thin film for surgery 100 of the embodiment of the present invention, a window 20 is provided on the thin film, the periphery of the window 20 is provided with an identifier, it is convenient to perform surgical operation by using the window 20, and the window 20 can be used as a virtual reality projection window 20 during the surgery. Combined with the positioning of the identification layer 12, a projection image of the surgical area can be displayed in real time, so that it is convenient for the camera to accurately locate the opening position of the patient according to the identifier, thereby solving the problem of line of sight blocking and positioning accuracy of the opening position. At the same time, by the setting of the identifier, more reliable data is provided for camera imaging, and a good foundation is established for the imaging system to better perform imaging on the intraoperative image. In addition, the structure of the present invention is simple, easy to produce and manufacture, and has low manufacturing cost.

Unless otherwise defined, the technical terms or scientific terms used in the present invention should be the common meaning understood by people with general skills in the art to which the present invention belongs. The words "first", "second" and similar words used in the present invention do not indicate any order, quantity or importance, but are only used to distinguish different components. The words "connected" or "connection" and similar words are not limited to physical or mechanical connections, but may include electrical connections, whether direct or indirect. "Up", "down", "left", "right" and the like are only used to indicate relative positional relationships, and when the absolute position of the described object changes, the relative positional relationship also changes accordingly.

The foregoing descriptions are preferred embodiments of the present invention. It should be noted that a person of ordinary skill in the art may make certain improvements and polishing without departing from the scope of the appended claims.

## Claims

1. A multifunctional thin film for surgery (100) by a surgical operator, comprising a thin film main body (10), wherein the thin film main body is provided with a window (20) for surgical operation inside a surgical opening of a patient, the thin film main body (10) is provided with at least three layers of structure (11, 12, 13) in a thickness direction, and the three layers of structure (11, 12, 13) comprise:
a thin film layer (11) made of a transparent plastic material;
an identification layer (12), wherein the identification layer is provided on a surface of the thin film layer (11), and is located at the periphery of the window (20), and the identification layer (12) is used to identify a position of the window (20) and basic information related to the surgery; and
an isolation paper layer (13), wherein the isolation paper layer (13) is provided on the surface of the identification layer (12) away from the thin film layer (11),
wherein the identification layer (12) is configured to be:
- fixed on a periphery of the surgical opening of the patient when being applied to a skin surface of the patient, and
- scanned by an imaging device combined with the multifunctional thin film to locate the position of the surgical opening, so that the surgical operator performs operations inside the surgical opening through the window (20),
and wherein the window (20) forms during surgery a virtual reality projection window, through which the imaging device projects an image (30) of the surgical opening in real time.

2. The multifunctional thin film for surgery (100) according to claim 1, wherein the identification layer (12) comprises:
a first identifier (121), wherein the first identifier (121) is provided in a first area of the identification layer (12), and is used to identify and manage the basic information related to the surgery; and
a second identifier (122), wherein the second identifier (122) is provided in a second area of the identification layer (12), and is used to identify position coordinates of the window (20).

3. The multifunctional thin film for surgery (100) according to claim 2, wherein the basic information related to the surgery comprises: the time of the surgery, the location of the surgery, and data information of preoperative and intraoperative tests of a patient.

4. The multifunctional thin film for surgery (100) according to claim 2, wherein the periphery of the window (20) comprises a top edge, a bottom edge opposite to the top edge, and two side edges respectively connecting two ends of the top edge and the bottom edge, the first identifier (121) is provided on the top edge; and the second identifier (122) is provided on at least two of the two side edges and the bottom edge.

5. The multifunctional thin film for surgery (100) according to any one of claims 2-4, wherein both the first identifier (121) and the second identifier (122) are two-dimensional barcode identifiers.

6. The multifunctional thin film for surgery (100) according to claim 5, wherein the two-dimensional barcode identifier is a QR code or an ArUco code.

7. The multifunctional thin film for surgery (100) according to claim 2, wherein a plurality of first identifiers (121) are provided, a plurality of second identifiers (122) are provided, and a spacing between the identifiers (121, 122) is greater than a preset spacing.

8. The multifunctional thin film for surgery (100) according to claim 7, wherein the preset spacing is 2 mm.

9. The multifunctional thin film for surgery (100) according to claim 2, wherein each first identifier (121) is provided with a corresponding first number according to a set position, and each second identifier (122) is provided with a corresponding second number according to a set position.

10. The multifunctional thin film for surgery (100) according to any one of claims 2 to 4, wherein the size of the first identifier (121) is larger than the size of the second identifier (122).

11. The multifunctional thin film for surgery (100) according to claim 9, wherein the thin film layer (11), the identification layer (12), and the isolation paper layer (13) are fixed by using an adhesive.

12. A surgery system for surgical operation inside a surgical opening of a patient by a surgical operator, the surgery system comprising:
(i) a multifunctional thin film for surgery (100) by the surgical operator according to claim 1; and
(ii) an imaging device which comprises a surgical robot and a camera and which is combined to the multifunctional thin film for surgery (100).

13. The surgery system according to claim 12, wherein the imaging device is configured to project a preoperative CT image or an intraoperative OCT image forming said image (30).

14. The surgery system according to claim 12 or 13, wherein the identification layer (12) comprises:
a first identifier (121), wherein the first identifier (121) is provided in a first area of the identification layer (12), and is used to identify and manage the basic information related to the surgery; and
a second identifier (122), wherein the second identifier (122) is provided in a second area of the identification layer (12), and is used to identify position coordinates of the window (20),
and wherein a precise positioning of the opening position is implemented after the imaging device recognizes the second identifier (122).

15. The surgery system according to claim 14, wherein both the first identifier (121) and the second identifier (122) are two-dimensional barcode identifiers.

## Patentansprüche

1. Multifunktionaler Dünnfilm für Operationen (100) durch einen Operateur, umfassend einen Dünnfilm-Hauptkörper (10), wobei der Dünnfilm-Hauptkörper mit einem Fenster (20) für die chirurgische Operation innerhalb einer Operationsöffnung eines Patienten versehen ist, wobei der Dünnfilm-Hauptkörper (10) mit mindestens drei Strukturschichten (11, 12, 13) in einer Dickenrichtung versehen ist und die drei Strukturschichten (11, 12, 13) Folgendes umfassen:
eine Dünnfilmschicht (11) aus einem transparenten Kunststoffmaterial;
eine Identifikationsschicht (12), wobei die Identifikationsschicht auf einer Oberfläche der Dünnfilmschicht (11) bereitgestellt ist und sich am Umfang des Fensters (20) befindet, und die Identifikationsschicht (12) verwendet wird, um eine Position des Fensters (20) und grundlegende Informationen im Zusammenhang mit der Operation zu identifizieren; und
eine Isolierpapierschicht (13), wobei die Isolierpapierschicht (13) auf der von der Dünnfilmschicht (11) abgewandten Oberfläche der Identifikationsschicht (12) vorgesehen ist,
wobei
die Identifikationsschicht (12) so ausgebildet ist, dass sie:
- an einem Umfang der Operationsöffnung des Patienten fixiert wird, wenn sie auf eine Hautoberfläche des Patienten aufgebracht wird, und
- von einer Bildgebungsvorrichtung in Kombination mit dem multifunktionalen Dünnfilm gescannt wird, um die Position der Operationsöffnung zu lokalisieren, sodass der Operateur Operationen innerhalb der Operationsöffnung durch das Fenster (20) durchführt, und wobei das Fenster (20) während der Operation ein Virtual-Reality-Projektionsfenster bildet, durch das die Bildgebungsvorrichtung ein Bild (30) der Operationsöffnung in Echtzeit projiziert.

2. Multifunktionaler Dünnfilm für Operationen (100) nach Anspruch 1, wobei die Identifikationsschicht (12) Folgendes umfasst:
einen ersten Identifikator (121), wobei der erste Identifikator (121) in einem ersten Bereich der Identifikationsschicht (12) bereitgestellt ist und verwendet wird, um die grundlegenden Informationen im Zusammenhang mit der Operation zu identifizieren und zu verwalten; und
einen zweiten Identifikator (122), wobei der zweite Identifikator (122) in einem zweiten Bereich der Identifikationsschicht (12) bereitgestellt ist und verwendet wird, um Positionskoordinaten des Fensters (20) zu identifizieren.

3. Multifunktionaler Dünnfilm für Operationen (100) nach Anspruch 2, wobei die grundlegenden Informationen im Zusammenhang mit der Operation Folgendes umfassen: den Zeitpunkt der Operation, den Ort der Operation und Dateninformationen von präoperativen und intraoperativen Tests eines Patienten.

4. Multifunktionaler Dünnfilm für Operationen (100) nach Anspruch 2, wobei der Umfang des Fensters (20) eine Oberkante, eine der Oberkante gegenüberliegende Unterkante und zwei Seitenkanten umfasst, die jeweils zwei Enden der Oberkante und der Unterkante verbinden, wobei der erste Identifikator (121) an der Oberkante vorgesehen ist; und der zweite Identifikator (122) an mindestens zwei von den beiden Seitenkanten und der Unterkante vorgesehen ist.

5. Multifunktionaler Dünnfilm für Operationen (100) nach einem der Ansprüche 2 bis 4, wobei sowohl der erste Identifikator (121) als auch der zweite Identifikator (122) zweidimensionale Barcode-Identifikatoren sind.

6. Multifunktionaler Dünnfilm für Operationen (100) nach Anspruch 5, wobei der zweidimensionale Barcode-Identifikator ein QR-Code oder ein ArUco-Code ist.

7. Multifunktionaler Dünnfilm für Operationen (100) nach Anspruch 2, wobei eine Vielzahl von ersten Identifikatoren (121) bereitgestellt sind, eine Vielzahl von zweiten Identifikatoren (122) bereitgestellt sind und ein Abstand zwischen den Identifikatoren (121, 122) größer als ein voreingestellter Abstand ist.

8. Multifunktionaler Dünnfilm für Operationen (100) nach Anspruch 7, wobei der voreingestellte Abstand 2 mm beträgt.

9. Multifunktionaler Dünnfilm für Operationen (100) nach Anspruch 2, wobei jeder erste Identifikator (121) mit einer entsprechenden ersten Zahl gemäß einer eingestellten Position versehen ist und jeder zweite Identifikator (122) mit einer entsprechenden zweiten Zahl gemäß einer eingestellten Position versehen ist.

10. Multifunktionaler Dünnfilm für Operationen (100) nach einem der Ansprüche 2 bis 4, wobei die Größe des ersten Identifikators (121) größer ist als die Größe des zweiten Identifikators (122).

11. Multifunktionaler Dünnfilm für Operationen (100) nach Anspruch 9, wobei die Dünnfilmschicht (11), die Identifikationsschicht (12) und die Isolierpapierschicht (13) durch Verwendung eines Klebstoffs fixiert werden.

12. Operationssystem für chirurgische Operationen innerhalb einer Operationsöffnung eines Patienten durch einen Operateur, wobei das Operationssystem Folgendes umfasst:
(i) einen multifunktionalen Dünnfilm für Operationen (100) durch den Operateur nach Anspruch 1;
(ii) eine Bildgebungsvorrichtung, die einen chirurgischen Roboter und eine Kamera umfasst und die mit dem multifunktionalen Dünnfilm für Operationen (100) kombiniert ist.

13. Operationssystem nach Anspruch 12, wobei die Bildgebungsvorrichtung so ausgebildet ist, dass sie ein präoperatives CT-Bild oder ein intraoperatives OCT-Bild projiziert, wodurch das Bild (30) gebildet wird.

14. Operationssystem nach Anspruch 12 oder 13, wobei die Identifikationsschicht (12) Folgendes umfasst:
einen ersten Identifikator (121), wobei der erste Identifikator (121) in einem ersten Bereich der Identifikationsschicht (12) bereitgestellt ist und verwendet wird, um die grundlegenden Informationen im Zusammenhang mit der Operation zu identifizieren und zu verwalten; und
einen zweiten Identifikator (122), wobei der zweite Identifikator (122) in einem zweiten Bereich der Identifikationsschicht (12) bereitgestellt ist und verwendet wird, um Positionskoordinaten des Fensters (20) zu identifizieren,
und wobei eine präzise Positionierung der Öffnungsposition implementiert wird, nachdem die Bildgebungsvorrichtung den zweiten Identifikator (122) erkannt hat.

15. Operationssystem nach Anspruch 14, wobei sowohl der erste Identifikator (121) als auch der zweite Identifikator (122) zweidimensionale Barcode-Identifikatoren sind.

## Revendications

1. Film mince multifonctionnel pour chirurgie (100) par un opérateur chirurgical, comportant un corps principal de film mince (10), dans lequel le corps principal de film mince est doté d'une fenêtre (20) pour intervention chirurgicale à l'intérieur d'une ouverture chirurgicale d'un patient, le corps principal de film mince (10) est doté d'au moins trois couches de structure (11, 12, 13) dans une direction d'épaisseur, et les trois couches de structure (11, 12, 13) comportent :
une couche de film mince (11) constituée d'un matériau plastique transparent ;
une couche d'identification (12), dans lequel la couche d'identification est prévue sur une surface de la couche de film mince (11), et est située à la périphérie de la fenêtre (20), et la couche d'identification (12) est utilisée pour identifier une position de la fenêtre (20) et des informations de base liées à la chirurgie ; et
une couche de papier isolant (13), dans lequel la couche de papier isolant (13) est prévue sur la surface de la couche d'identification (12) à l'écart de la couche de film mince (11),
dans lequel
la couche d'identification (12) est configurée pour être :
- fixée sur une périphérie de l'ouverture chirurgicale du patient lorsqu'elle est appliquée sur une surface cutanée du patient, et
- balayée par un dispositif d'imagerie combiné avec le film mince multifonctionnel pour localiser la position de l'ouverture chirurgicale, de sorte que l'opérateur chirurgical réalise des opérations à l'intérieur de l'ouverture chirurgicale à travers la fenêtre (20), et dans lequel la fenêtre (20) forme pendant la chirurgie une fenêtre de projection de réalité virtuelle, à travers laquelle le dispositif d'imagerie projette une image (30) de l'ouverture chirurgicale en temps réel.

2. Film mince multifonctionnel pour chirurgie (100) selon la revendication 1, dans lequel la couche d'identification (12) comporte :
un premier identifiant (121), dans lequel le premier identifiant (121) est prévu dans une première zone de la couche d'identification (12) et est utilisé pour identifier et gérer les informations de base relatives à la chirurgie ; et
un deuxième identifiant (122), dans lequel le deuxième identifiant (122) est prévu dans une deuxième zone de la couche d'identification (12) et est utilisé pour identifier les coordonnées de position de la fenêtre (20).

3. Film mince multifonctionnel pour chirurgie (100) selon la revendication 2, dans lequel les informations de base relatives à la chirurgie comportent : l'heure de la chirurgie, le lieu de la chirurgie et des informations de données de tests préopératoires et peropératoires d'un patient.

4. Film mince multifonctionnel pour chirurgie (100) selon la revendication 2, dans lequel la périphérie de la fenêtre (20) comporte un bord supérieur, un bord inférieur opposé au bord supérieur et deux bords latéraux reliant respectivement deux extrémités du bord supérieur et du bord inférieur, le premier identifiant (121) est prévu sur le bord supérieur ; et le deuxième identifiant (122) est prévu sur au moins deux des deux bords latéraux et du bord inférieur.

5. Film mince multifonctionnel pour chirurgie (100) selon l'une quelconque des revendications 2 à 4, dans lequel le premier identifiant (121) et le deuxième identifiant (122) sont tous deux des identifiants de codes-barres bidimensionnels.

6. Film mince multifonctionnel pour chirurgie (100) selon la revendication 5, dans lequel l'identifiant de code-barres bidimensionnel est un code QR ou un code ArUco.

7. Film mince multifonctionnel pour chirurgie (100) selon la revendication 2, dans lequel une pluralité de premiers identifiants (121) sont prévus, une pluralité de deuxièmes identifiants (122) sont prévus et un espacement entre les identifiants (121, 122) est supérieur à un espacement prédéfini.

8. Film mince multifonctionnel pour chirurgie (100) selon la revendication 7, dans lequel l'espacement prédéfini est de 2 mm.

9. Film mince multifonctionnel pour chirurgie (100) selon la revendication 2, dans lequel chaque premier identifiant (121) est doté d'un premier numéro correspondant selon une position définie, et chaque deuxième identifiant (122) est doté d'un deuxième numéro correspondant selon une position définie.

10. Film mince multifonctionnel pour chirurgie (100) selon l'une quelconque des revendications 2 à 4, dans lequel la taille du premier identifiant (121) est supérieure à la taille du deuxième identifiant (122).

11. Film mince multifonctionnel pour chirurgie (100) selon la revendication 9, dans lequel la couche de film mince (11), la couche d'identification (12) et la couche de papier isolant (13) sont fixées en utilisant un adhésif.

12. Système chirurgical pour opération chirurgicale à l'intérieur d'une ouverture chirurgicale d'un patient par un opérateur chirurgical, le système chirurgical comportant :
(i) un film mince multifonctionnel pour chirurgie (100) par l'opérateur chirurgical selon la revendication 1 ;
(ii) un dispositif d'imagerie qui comporte un robot chirurgical et une caméra et qui est combiné avec le film mince multifonctionnel pour chirurgie (100).

13. Système chirurgical selon la revendication 12, dans lequel le dispositif d'imagerie est configuré pour projeter une image CT préopératoire ou une image OCT peropératoire formant ladite image (30).

14. Système chirurgical selon la revendication 12 ou 13, dans lequel la couche d'identification (12) comporte :
un premier identifiant (121), dans lequel le premier identifiant (121) est prévu dans une première zone de la couche d'identification (12) et est utilisé pour identifier et gérer les informations de base relatives à la chirurgie ; et
un deuxième identifiant (122), dans lequel le deuxième identifiant (122) est prévu dans une deuxième zone de la couche d'identification (12) et est utilisé pour identifier les coordonnées de position de la fenêtre (20),
et dans lequel un positionnement précis de la position d'ouverture est mis en œuvre après que le dispositif d'imagerie reconnaît le deuxième identifiant (122).

15. Système chirurgical selon la revendication 14, dans lequel le premier identifiant (121) et le deuxième identifiant (122) sont tous deux des identifiants de codes-barres bidimensionnels.
